# EUROPEAN PATENT APPLICATION

(11) **EP 3 639 776 A1**
(43) Date of publication of application: **22.04.2020**
(21) Application number: 19201677.2
(22) Date of filing: 08.07.2015
(51) Int. Cl.: A61B 17/80, A61B 17/064, A61B 17/068, A61B 17/70, A61B 17/17, A61B 17/88, A61B 17/00

(54) **BONE IMPLANT WITH ANTI-ROTATION**

(30) Priority: 10.07.2014 US 201462022811 P; 12.08.2014 US 201462036240 P
(62) Divisional of application: 15818535.5
(71) Applicant: Crossroads Extremity Systems, LLC, Memphis TN 38119-5702 (US)
(72) Inventor: COLEMAN, Glen, Cordova, Tennessee 38018 (US); HARTDEGEN, Vernon, Collierville, Tennessee 38017 (US); HOLLIS, Michael, Collierville, Tennessee 38017 (US)
(74) Representative: HGF Limited

(57) **Abstract**

A medical implant for osteosynthesis wherein the implant has a first configuration when it is releasably attached to a delivery instrument and a second configuration when it is released from the delivery instrument. The implant has non-symmetrical means for fixation to bone segments and when the implant is released following insertion into the bone segments the fixation means prevent or minimizes rotation or other movement of the bone segments relative one another. The fixation means also have means to prevent pullout from bone. The means for fixation may be adapted to compress, distract or control special orientation of the bone segments relative to one another. The implant may be packaged in a kit with tools to facilitate implant surgery.

## Description

### BACKGROUND OF THE INVENTION

### Field of the Invention

The present invention is in the technical field of medical devices. More particularly, the present invention is in the technical field of bone fixation or arthrodesis or deformity correction. The invention relates to a fixation system for bones of all types utilizing an implant. Such systems are used in osteosynthesis (bone fusion), wherein the implant bridges the fracture generating a force (typically compression) across the bone members. The force is generated by either the properties of the implant, the different configurations of the implant, the surgical technique or placement of the implant or a combination thereof. For example, the implant may have a first configuration when in free-state and a second configuration required for insertion. It may be desirable for optimal implant placement and function to be able to pre-assemble or attach the implant to an inserter to facilitate placement of the implant on or in the bone. Once implanted, it is desirable to prevent or minimize rotation or other movement of the bone segments relative to one another. The implant may be indicated for the various bones of the entire skeleton.

### The Related Art

The present invention seeks to remedy the problems of the prior art. The invention produces a system that allows placement of an implant in its final required position with or without additional manipulation. In addition, the present invention will provide resistance to pullout and/or rotation once implanted. The implant features may be particularly beneficial in a compression implant. The current invention may or may not rely on additional implant positioning once positioned in the bone. Also, the current invention may incorporate other necessary features for delivery of the implant into the bone.

### SUMMARY OF THE INVENTION

The present invention includes an implant or other bone fastening device. The implant may be a bone staple, bone plate, modular staple, or the like. The implant has elastic properties or other material properties that allow the device to have at least two configurations or configurable to various positions when placed on or in the bone. The free-state or implanted-state of the device provides a force, typically compression, across two or more bone members. An inserter is used to hold the implant or other fastening device in a configuration that is different than the free-state or implanted-state configuration. This first configuration may be useful in placement of the implant onto or into bone members. Fastening device and implant are used interchangeably in this application and are not intended to be limiting in nature.

The present invention may have implant legs that once inserted into bone provide a dynamic compression and prevent pullout and/or rotation of the adjoining bone segment. The implant also has features for releasably engaging an implant inserter. The present invention includes an implant or portion of an implant that is made of an elastic material or a material that may allow the implant to have multiple configurations. The ability of the implant to have multiple configurations may be achieved by the material properties that have shape memory or super elastic properties or it may be achieved by manipulation (mechanical, physical, chemical, temperature, electrical or otherwise) of the implant to create a second configuration. The implant is held in one configuration during insertion or removal and returns to or is placed in another configuration in its free-state or implanted-state. The implant may have multiple configurations, for example one for inserting into the bone and at least a second configuration for compressing, distracting, controlling spatial orientation or the like of one or more bone segments.

The implant has features for engaging the bone. These features may include bone screws, leg members, pins or other features for attaching the implant to bone. The implant may have one or more bridge members. The implant may have leg members for engaging the bone. The implant may have modular members for engaging bone, such as bone screws or pegs. To those skilled in the art, based on the description of the invention herein, it will be evident that multiple options exist for connecting an implant to bone. The connecting members or features may not necessarily be of the same material as the bridge component. The deformability aspect of the current invention may be in the bridge member(s), the connecting member(s) or another member(s) of the implant or fixation device. The leg member(s) may be configured to receive members from the inserter to hold the implant in its first configuration or to allow manipulation of the implant to another configuration. The leg members also have features or geometry to prevent relative movement between two adjoining bone segments. For example, these features may include fins, projections, non-circular geometries or the like that may prevent rotation of the leg within each bone segment thereby preventing rotation between the adjoining bone segments.

A "bone fixation device" or implant may include any of a variety of devices that secure an object to a bone, including but not limited to staples, bone plates, modular staples, bone screws, pins, blades, suture anchors, and the like. The terms "inserter," "implant inserter," "insertion device," and "delivery instrument" are used herein interchangeably.

### BRIEF DESCRIPTION OF THE DRAWINGS

Figure 1 is a perspective view of a first embodiment of the present invention.
Figure 2 is a front view of a first embodiment of the present invention further showing the location of section A-A.
Figure 3 is the section view A-A of the leg of the first embodiment shown in Figure 2.
Figure 4 is a front view of a second embodiment of the present invention showing alternate leg geometry and a section B-B.
Figure 5 is the section view B-B of the second embodiment shown in Figure 4.
Figure 6 depicts other possible cross sections for other embodiments of the staple legs.
Figure 7 is a perspective view of a third embodiment of the current invention.
Figure 8 is a perspective view of a fourth embodiment of the current invention.
Figure 9 is a top view of an implant kit that may be provided for inserting the implant of the current invention into bone segments.
Figure 10a shows a section view of two bones with an exemplary embodiment of the current invention partially inserted into the bones.
Figure 10b shows a section view of two bones with an exemplary embodiment of the current invention fully inserted into the bones.

### DETAILED DESCRIPTION OF THE INVENTION

The present invention includes an implant for spanning and/or fixating at least two bone segments. The exemplary embodiments of the current invention are discussed in the description of the figures below. The implant may be of a configuration similar to a bone staple as discussed below. The present invention includes an apparatus or instrument for inserting the device that may be pre-assembled or affixed to the implant. The implant or implants could be held in a particular configuration prior to use that facilitates insertion into the bone segments. The embodiments described herein may be used in connection with any type of inserter or fixation device that is compatible with the description and objectives stated herein, including but not limited to various bone staples, bone plates, etc. that may have more than one implant configuration and may generate a force, typically a compressive force, across bone segments.

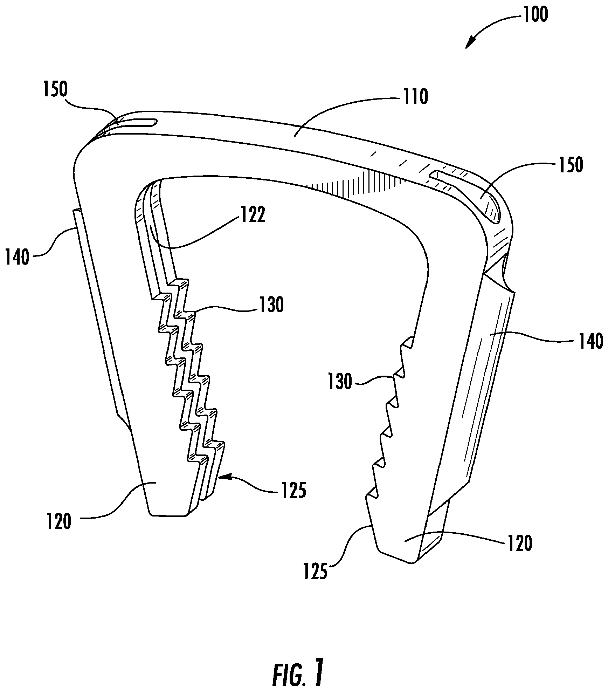

Figure 1 shows a bone implant 100 consisting of a bridge 110 and legs 120. The implant 100 shown in Figure 1 may have legs 120 converging or in a compressed state. This may be the free-state or implanted state of the implant 100. Bridge member 110 may be of uniform cross section or of a varying cross section as depicted in Figure 1. In this particular embodiment bridge member 110 consist of an arched geometry. Legs 120 may have a tapered tip 125 that may facilitate insertion into the bone. Legs 120 may also have barbs 130 that may minimize or prevent pullout and/or rotation. In this exemplary embodiment, legs 120 have barbs 130 on either side of groove 122. This interrupted barb geometry may further resist rotation of the leg 120 in a bone. The barbs 130 are shown on the internal surface of this embodiment, but may also be present on an external surface of the legs 120. The implant 100 has fin like projections 140 to resist rotation of the legs 120 within the bone thereby resisting rotation between the bone segments in which the legs 120 are implanted. In this embodiment the fin 140 is shown on one external surface of the implant leg 120. The fin 140 may also be on more than one surface of leg 120 and not necessarily on the surface directly opposed to the barbs 130. Implant 100 has opening 150 for attachment to an inserter or to allow manufacturing of the non-symmetrical legs 120 and particularly groove 122. As shown in Figure 1, staple legs 120 may have a groove 122 that is defined by the shape of the opening 150 and the outer geometry of the legs 120. The non-circular geometry of the legs 120 when inserted into the bone provides an interference type fit with the bone such that the non-circular geometry will not rotate within the bone. Leg 120 should not rotate within the bone because the non-circular and/or non-symmetrical geometry does not match the prepared hole in the bone. An interference fit within the bone may be generated by using a drill that has a smaller diameter than the circumscribed outer diameter of the implant leg 120 or the prepared hole is of a different geometry than the implant leg 120. Leg 120 may not rotate within the bone because the non-circular and/or non-symmetrical geometry may create a "keyed" interlock with the bone in which the leg is inserted. This keyed geometry may be the negative of the implant leg geometry.

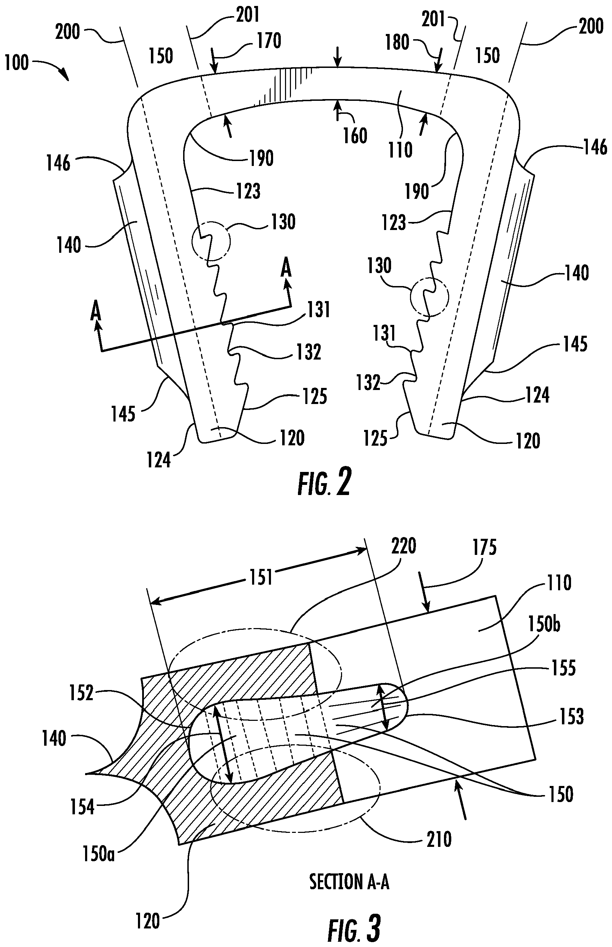

Figure 2 is a front view of implant 100 showing bridge component 110, legs 120, fins 140 and barbs 130. Bridge member 110 is shown with an arched geometry of varying thickness. Bridge member 110 has a center thickness 160 and two outer thicknesses 170 and 180 at the connection region of the bridge 110 to the legs 120. This connection region may be further defined by joint 190 which may be arched, gusseted or some other suitable geometry. In this embodiment, thickness 160 is less than thickness 170 and 180. Thickness 170 and 180 are shown as being equivalent in this particular embodiment. Thickness 160 may be more or less than thickness 170 and thickness 180. Thickness 170 may be more or less than thickness 180. The combination of geometries 160, 170, 180 and 190 are such that the stresses seen by the bridge member 110 and connection region of the bridge member 110 and legs 120 are sufficient to withstand the loading environment of the implant. The legs 120 are shown to have barbs 130. The barbs may have peaks 131 and valleys 132. The barbs 130 may have multiple geometries and configurations. The barbs may or may not be present or needed in a particular embodiment. The merits of the current invention are still viable in the absence of the barbs 130. The number of barbs 130 may vary depending on the length of the leg 120. As shown in this embodiment, the barb peaks 131 are proud to surface 123. The barb valleys 132 are relatively equal to the surface 123. In an alternate embodiment the barb peaks 131 may be below surface 123 or at the level of surface 123. Still further in an alternate embodiment, barb valleys 132 may be below surface 123 or above surface 123. Any combination of the location of the barb peaks 131 and barb valleys 132 relative to surface 123 may be possible. The fin 140 is located on the external leg surface 124. The fin 140 may be proud or extend beyond surface 124. Fin 140 may include a lead-in surface 145 to facilitate insertion into a bone segment. Fin 140 may also include a surface 146 that may sit below a bone surface to prevent or resist pullout of the staple leg 120. Fin 140 may include barbs similar to the barb 130. Fin 140 is of a geometry that creates a non-circular and/or non-symmetrical staple leg such that the geometry of the staple leg 120 will resist rotation within a bone segment. The geometry of the staple leg 120 in combination with the fin 140 and groove 122 may not match the geometry of the prepared hole in the bone for which the staple leg 120 will be inserted. This may create an interference fit between the staple leg 120 and the prepared hole. The implant 100 may be inserted into a bone without preparing a hole in the bone segment. The geometry of the staple leg 120 in combination with the fin 140 and groove 122 may create an interlock between the bone geometry and the implant leg geometry 120 in an unprepared bone segment.

Figure 2 includes dashed lines 200 and 201 to represent opening 150 as also illustrated in Figure 1. Opening 150 is used for attachment to a delivery instrument or inserter. Opening 150 may be used to create groove 122. As shown in Figure 2, opening 150 is positioned such that the opening extends through the top of the bridge 110 to the bottom of the staple legs 120. The opening 150 opens toward the inside of the staple leg 120 creating the groove 122. In an alternate embodiment, groove 122 may open toward the outside of the legs.

Opening 150 in Figure 2 is positioned such that the opening 150 spans the connecting region between bridge 110 and the leg 120 which may include radius 190 thereby opening to the internal region of the staple (i.e. towards the midline of the staple 100). The inner border, 201 of the opening 150 may extend into the bridge member 110. The outer border 200 of opening 150 may extend through the entire length of the staple leg 120. The position of the inner border 201 may be advantageous in creating groove 122 and the non-circular, non-symmetrical geometry of the staple leg 120. The position of the inner border 201 may be advantageous in the manufacturing of the implant 100, particularly the geometry of the leg 120, which may include the groove 122. This positioning of the inner border 201 of opening 150 may allow ease of manufacturing by providing a thinner section in the area of thickness 170 or 180 to, for example, provide a starter hole for a wire EDM process or laser cut process or machining process. The present invention may include the positioning of opening 150. The positioning of opening 150 may be such that it extends through a thinner section of the implant, in this embodiment the thickness 170 of the implant bridge 110. This may be particularly advantageous in the setup of the manufacturing process. Having the inner border 201 extending through the thinner section 170 may allow the manufacture a simplified more cost effective manufacturing process without having to create groove 122 entirely within the perimeter of the staple leg. The position of opening 150 relative to the staple leg 120 and/or the bridge member 110 and/or joint region 190 may provide a means for distributing the required stresses within the implant to achieve a desired compressive force. The position and geometry of opening 150 may be altered within the scope of this invention to manipulate the stress distribution to achieve the desired performance. Within the scope of this invention, the position and geometry of opening 150 may be altered in combination with the thicknesses 170, 160, 180 and/or joint 190 to manipulate the stress distribution to achieve the desired performance. The amount of desired force generated by the current invention may be accomplished by manipulating individual aspects of the implant geometry and their relative orientations to one another. The geometry and relative orientation of thicknesses 160, 170 and/or 180 may be altered to manipulate the stress distribution within the implant bridge 110 to achieve the desired performance. The geometry and relative orientation of thicknesses 160, 170 and/or 180 may be altered in combination with the joint region 190 geometry and relative orientation to manipulate the stress distribution within the implant bridge 110 and/or implant legs 120 to achieve the desired performance. The geometry and relative orientation of thicknesses 160, 170 and/or 180 may be altered in combination with the joint region 190 geometry and relative orientation and may be in combination with altering the geometry and relative orientation of opening 150 to manipulate the stress distribution within the implant bridge 110 and/or implant legs 120 to achieve the desired performance. The geometry and relative orientation of opening 150 may be altered to manipulate the stress distribution within the implant bridge 110 and/or implant legs 120 to achieve the desired stress distribution and/or performance. Furthermore, the implant width 175 as shown in Figure 3 may also be manipulated in geometry and/or orientation either as an independent variable or in combination with the previously described aspect or aspects of the implant to manipulate the stress distribution within the implant bridge 110 and/or implant legs 120 to achieve the desired performance or force generation.

Figure 3 shows the cross section view A-A as noted in Figure 2 illustrating that the cross section of the opening does not need to be uniform, although a uniform cross section can optionally be used. Opening 150 is further divided into openings 150a and 150b. Opening 150a is represented by the vertical dashed lines in Figure 3 and may be bounded by the portion of opening 150 surrounded by the staple leg 120 and opening toward the midline of the implant 100. This portion 150a of the opening 150 is analogous to the previously discussed groove 122. Opening 150b is represented by the horizontal solid lines in Figure 3 and may be bounded by the portion of opening 150 contained in the staple bridge 110 and inside edge of the staple leg 120. Combined, opening 150a and 150b make up opening 150. Opening 150 may extend out of one particular side of a staple leg or may be contained completely within a staple leg or completely outside a staple leg. Opening 150 has an overall length 151 and may include an outside width 154 and an inside width 155. Width 154 and 155 may or may not be equal in size. Length 151 is greater than lengths 154 and 155. Width 155 may be more or less than width 154. In this exemplary embodiment opening 150 may have an outer edge generated by a radius 152 and an inner edge generated by radius 153. Radius 152 may or may not be the same as 153. Radius 153 may be greater than or less than 152. The outer borders of opening 150 may or may not be radii. Geometries, such as tear drops, squares, rectangles, ovals, slots, keyholes, etc. may be used to create opening 150. Opening 150 is such that the geometry is more resistant to bending in the direction of the force generated by the implant. In this particular embodiment, space 150 is more resistant to bending in the direction of the compressive force. Space 150 is used to facilitate insertion of the implant or connection to an inserter for implantation. The geometry of space 150 would mimic that geometry of the corresponding insertion tool. The insertion tool would have a geometry similar to that of space 150.

As shown in the exemplary embodiment of Figure 3, the interrupted staple leg geometry 120 may create a geometry that produces multiple fins or features to resist rotation. In this exemplary embodiment fin 140 exists on the outside perimeter of the staple leg 120. In addition other fins may be created in the outer geometry of the staple leg 120 and the groove 122, e.g. space 150a. As shown in Figure 3 the combination of the staple leg geometry 120, the opening 150 and/or groove 122 may be used to make an effective fin 210 and/or 220.

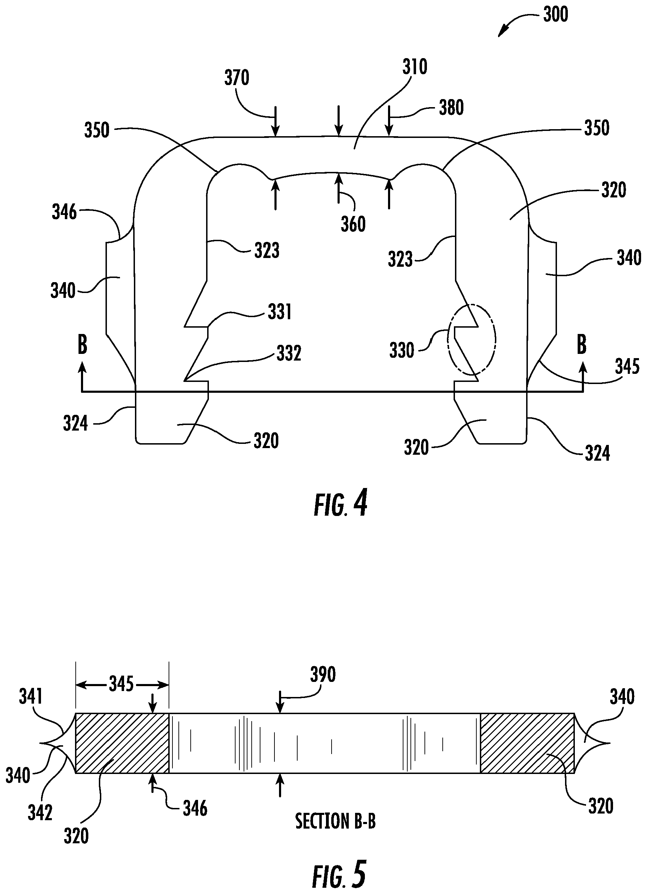

Figure 4 shows an embodiment of the current invention of a staple 300 having a staple bridge 310 and staple legs 320. In this particular embodiment the staple legs 320 are shown parallel relative to each other. Staple bridge 310 may be relatively flat across the top and may have a varying thickness represented by thickness 360, 370 and 380. Bridge member 310 has a center thickness 360 and two outer thicknesses 370 and 380. In this embodiment, thickness 360 is less than thickness 370 and 380. Thickness 370 and 380 are shown as being equivalent in this particular embodiment. Thickness 360 may be more or less than thickness 370 and thickness 380. Thickness 370 may be more or less than thickness 380. The connection region between the staple leg 320 and the staple bridge 310 has geometry 350. This geometry 350 may be used to facilitate insertion of the implant 300 or connection to an inserter for implantation. This geometry 350 may be arched, gusseted or some other suitable geometry. The combination of geometries 360, 370, 380 and 350 are such that the stresses seen by the bridge member 310 and connection region of the bridge member 310 and legs 320 are sufficient to withstand the loading environment of the implant. The position of geometry 350 relative to the staple leg 320 and/or the bridge member 310 may provide a means for distributing the required stresses with the implant to achieve a desired compressive force. The position and geometry of 350 may be altered within the scope of this invention to manipulate the stress distribution to achieve the desired performance. Within the scope of this invention, the position and geometry of undercut 350 may be altered in combination with the thicknesses 370, 360, and/or to manipulate the stress distribution to achieve the desired performance. The amount of desired force generated by the current invention may be accomplished by manipulating individual aspects of the implant geometry and their relative orientations to one another. The geometry and relative orientation of thicknesses 360, 370 and/or 380 may be altered to manipulate the stress distribution within the implant bridge 310 to achieve the desired performance. The geometry and relative orientation of thicknesses 360, 370 and/or 380 may be altered in combination with the joint region 350 geometry and relative orientation to manipulate the stress distribution within the implant bridge 310 and/or implant legs 320 to achieve the desired performance. The geometry and relative orientation of geometry 350 may be altered to manipulate the stress distribution within the implant bridge 310 and/or implant legs 320 to achieve the desired stress distribution and/or performance. Furthermore, the implant width 390 as shown in Figure 5 may also be manipulated in geometry and/or orientation either as an independent variable or in combination with the previously described aspect or aspects of the implant to manipulate the stress distribution within the implant bridge 310 and/or implant legs 320 to achieve the desired performance or force generation.

The legs 320 are shown to have barbs 330. The barbs may have peaks 331 and valleys 332. The barbs 330 may have multiple geometries and configurations. The barbs may or may not be present or needed in a particular embodiment. The merits of the current invention are still viable in the absence of the barbs 330. The number of barbs 330 may vary depending on the length of the leg 320. As shown in this embodiment, the barb peaks 331 are co-linear with the surface 323. The barb valleys 132 are below the surface 323. In an alternate embodiment the barb peaks 331 may be below surface 323 or above the level of surface 323. Still further in an alternate embodiment, barb valleys 332 may be below surface 323 or above surface 323. Any combination of the location of the barb peaks 331 and barb valleys 332 relative to surface 323 may be possible. The fin 340 is located on the external leg surface 324. The fin 340 may be proud or extend beyond surface 324. Fin 340 may include lead-in surface 345 to facilitate insertion into a bone segment. Fin 340 may also include a surface 346 that may sit below a bone surface to prevent or resist pullout of the staple leg 320. Fin 340 may include barbs similar to the barb 330. Fin 340 is of a geometry that creates a non-circular and/or non-symmetrical staple leg 320 such that the geometry of the staple leg 320 will resist rotation within a bone segment. The geometry of the staple leg 320 in combination with the fin 340 may not match the geometry of the prepared hole in the bone for which the staple leg 320 will be inserted. This may create an interference fit between the staple leg 320 and the prepared hole. The implant 300 may be inserted into a bone without preparing a hole in the bone segment. The geometry of the staple leg 320 in combination with the fin 340 may create an interlock between the bone geometry and the implant leg geometry 320 in an unprepared bone segment.

Figure 5 is the section view B-B as depicted in Figure 4. The staple leg 320 may be of solid construction without a groove. The staple leg 320 may have fin 340 protruding from a surface of the staple leg creating a non-symmetrical and/or non-circular geometry. Staple leg 320 may have a width 345 and a height 346. In this particular embodiment the staple leg height 346 is represented as being relatively equivalent to the staple bridge height 390. In other embodiments, staple leg height 346 may be more or less than staple bridge height 390. Figure 5 represents an embodiment that does not include and opening passing through the staple bridge 310 or the staple leg 320.

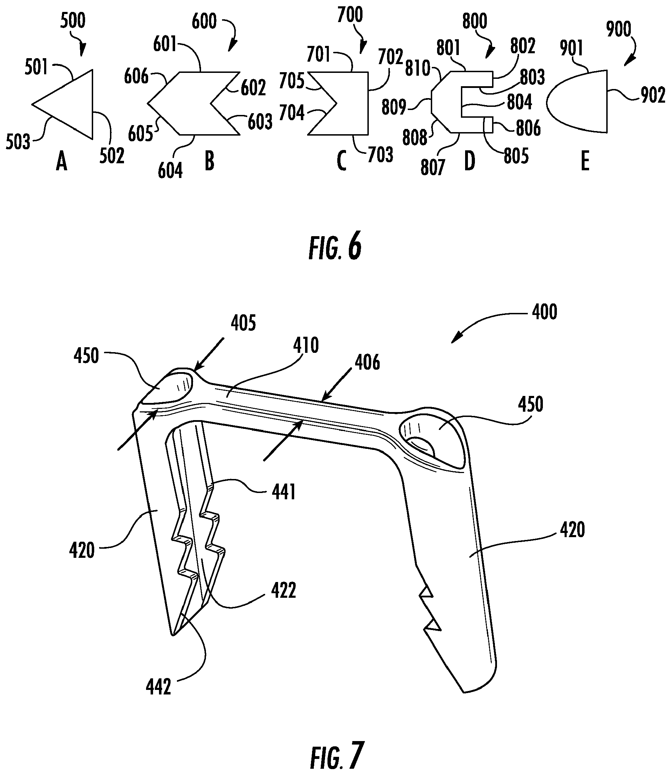

Figure 6 shows other possible cross-section geometries that may be used for staple legs 120 and/or 320. The embodiments shown in Figure 6 have various faces that create a geometry that is non-circular and/or non-symmetrical in at least one plane. The geometry of the staple legs in combination with a fin and/or a groove may not match the geometry of the prepared hole in the bone for which the staple leg will be inserted. This may create an interference fit between the staple leg and the prepared hole. The implant may be inserted into a bone without preparing a receiving hole in the bone segment. The geometry of the staple leg in combination with the fin and/or groove may create an interlock between the bone geometry and the implant leg geometry in an unprepared bone segment. With the current invention, it is also possible to create a broached hole in a bone segment that may match the non-circular and/or non-symmetrical geometry of a staple leg. The combination of the non-circular and/or non-symmetrical leg with a broach of similar geometry would resist rotation of the staple leg in the bone segment.

Figure 7 shows yet another embodiment of the current invention of staple 400 having a bridge 410 and legs 420. In this embodiment the width 405 of the legs 420 is different than the width 406 of the staple bridge 410. Opening 450 is present and extends into the bridge 410. This embodiment shows groove 422 extending the length of the staple leg 420. The combination of the geometry of the staple leg 420, the opening 450, the position of the opening 450 relative to the staple bridge 410 and the groove 422 creates effective fins 441 and 442.

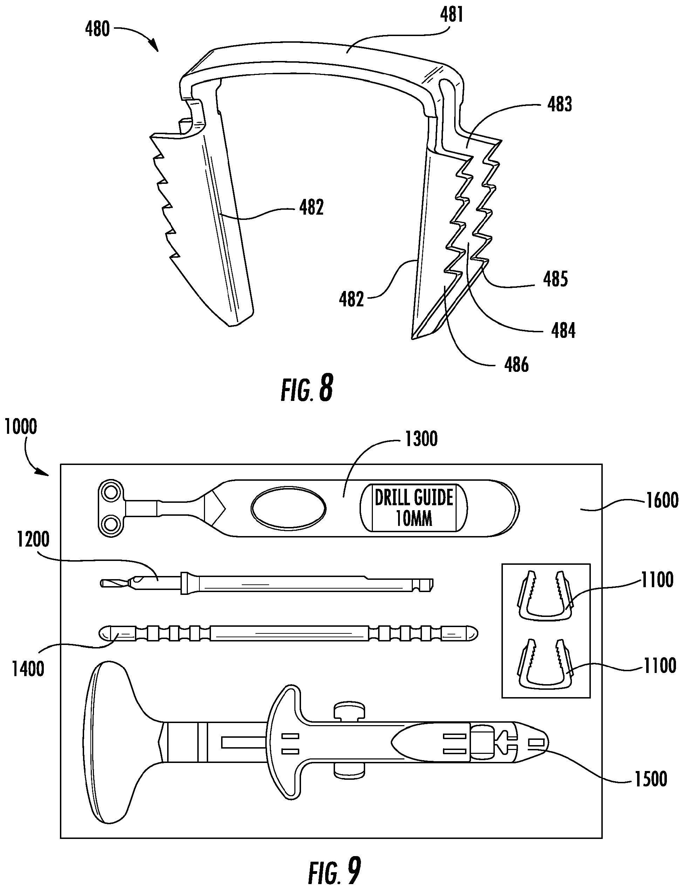

Figure 8 is yet another embodiment of the current invention, staple 480. Staple 480 has a bridge 481 and legs 482. The opening 483 is present but may not extend into the bridge 481. Opening 483 may open to the outside of the staple leg 482 creating groove 484. The combination of the geometry of the staple leg 482, the opening 483, the position of the opening 483 relative to the border of the staple leg and the groove 484 creates effective fins 485 and 486.

The implant of the current invention may be packaged as an implant kit with the associated instruments needed for a successful implantation. Such a kit may include the implant or implants, the necessary drills, reamers or broaches for preparing the bone for receiving the implant, any necessary drill guides and an inserter for facilitating implantation of the implant into the bone. This kit may be provided sterile packaged for single use. Figure 9 shows one embodiment of an implant kit 1000 that may be used to provide the end user with the necessary associated instruments for successfully implanting an implant of the current invention. Such a kit may include one or more implants 1100 similar to the embodiments described herein. The kit may also include a drill 1200, a drill guide 1300 and a provisional fixation pin 1400. The kit may also include an insertion tool or inserter 1500 for facilitating insertion of the implant 1100 into a bone segment. One embodiment of the kit may have the implant 1100 preassembled to the insertion tool 1500. The components of this kit may be assembled in a tray 1600 constructed of appropriate materials. Once the end user opens the kit, the surgical technique may include the following steps. After exposure of the operative site, the osteotomy or fracture may be reduced and held in place. The drill guide or reamer guide may be placed across the fusion site with both guide tubes against the bone. The first hole is drilled to final depth by advancing the drill, reamer or broach until the depth stop hits the top of the guide. A provisional fixation pin may be placed in the prepared hole to help maintain reduction while the second hole is prepared. Once both holes have been prepared, the legs of the implant, which has been assembled or loaded to the inserter tool, may be inserted into the prepared holes in the bone. Since an interference fit may be used, light tapping on the end of the inserter tool may be helpful in advancing the implant into the bone. The implant should be fully inserted until flush against the surface of the bone. The implant may then be released from the inserter tool. Alternatively, the holes in the bone may not be prepared for receiving the legs of the staple. As described herein, certain features of the current invention may be used or beneficial in a technique that does not require preparation of holes in the bone prior to insertion of the implant.

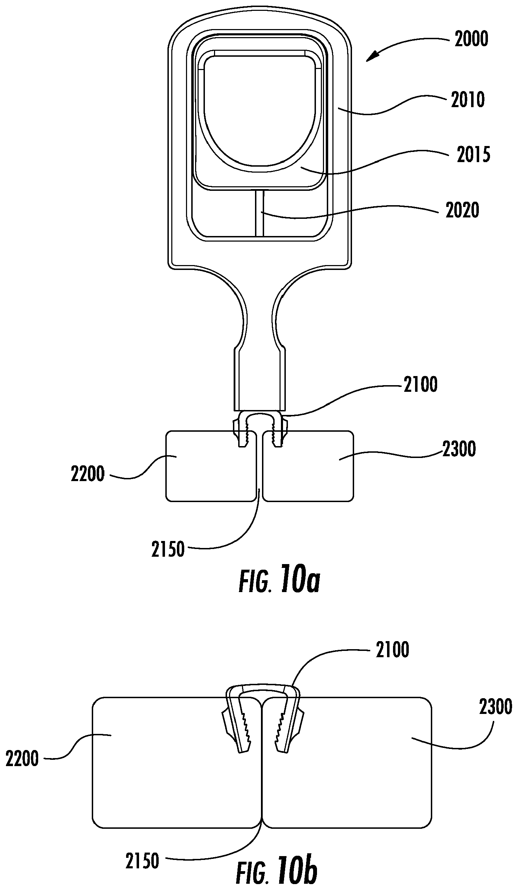

Figure 10a shows an implant 2100 of the current invention assembled to an inserter 2000 of the current invention partially inserted into bones 2200 and 2300. The inserter 2000 may have an inserter body 2010, an inserter plunger 2015 and an ejector pin 2020. The inserter has means for engaging the implant that allow the implant to be positioned flush to bone. For example, this embodiment would have inserter legs of a geometry that would mimic the space 150 as depicted in Figure 3. The inserter legs may be in a fixed orientation. In this embodiment the legs are in a fixed parallel orientation for insertion into the implant 2100. As the inserter legs are placed into the staple 2100, the staple legs are made parallel. The inserter plunger 2015 may push the ejector pin 2020 thereby releasing, ejecting or disengaging the implant 2100 from the inserter body 2010. Figure 10a shows the implant 2100 having parallel legs partially inserted in bones 2200 and 2300 showing the fusion site 2150 having a gap. The legs of implant 2100 are made relatively parallel and are maintained relatively parallel by the inserter 2000 to facilitate insertion into the bones 2200 and 2300. Once the implant 2100 is released from the inserter 2000 as shown in Figure 10b the legs may converge or compress drawing bones 2200 and 2300 toward one another causing the fusion site 2150 to compress with no gap.

The previous description of the embodiments described herein may be manufactured from a number of materials including those with elastic properties, such as super elastic nitinol. However this description is not intended to be limited by the materials of construction. Those skilled in the art will understand, based on the description of the invention herein, that the same may be accomplished using a material for example with shape memory aspects, such as shape memory nitinol or other materials that currently exist or may exist that have desirable material properties that will achieve the intended function of the current invention, for example stainless steel and/or titanium or titanium alloys. The use of an inserter/holding device may or may not be optional. The specific details of the inserter may vary greatly depending of the chosen embodiment. The exemplary embodiments described herein describe a one-piece staple-like implant. Those skilled in the art will understand, based on the description of the invention herein that an implant of multiple components would still be within the scope of the current invention. Other embodiments may include two or more fixation means that may be of the same style (such as bone screws, bone pegs, blades, staple legs, etc.) or of varying styles or some combination thereof. For fixation means that are modular in nature, they may or may not be made of the same material as the implant described herein. The embodiments described herein contemplate that both staple legs will include the same features. However, it is within the scope of the current invention that staple legs within the same implant may vary in their geometry, fins, length, cross section, barbs, or other physical characteristics relative to each other. Furthermore, one or more of the staple legs may be of a circular or symmetrical geometry while one or more of the other staple legs may be within the scope of this invention.

The exemplary embodiments described herein are not intended to be limiting. The embodiments described herein can be manufactured from a number of different materials or combinations of materials. In the exemplary embodiments described herein, the implant may be made of a material that may have elastic or spring properties or shape memory properties that may allow the implant to have more than one configuration. Nitinol, for example, possesses material properties, such as shape memory and/or super elasticity that may provide the inherent properties to allow an embodiment to have multiple configurations. Still other materials such as PEEK or other polymers may also possess material properties beneficial for the embodiments as described herein. A combination of materials may also be preferred. The scope of this invention would apply to staples or implants manufactured from a number of materials such as nitinol, stainless steel, titanium, PEEK, polymers, biologic or resorbable materials. Based on the description of the invention herein, those skilled in the art will realize the merits of the current invention are independent of material but may be more beneficial in some materials than others. For example, certain aspects of the invention may be more appealing in nitinol given its dynamic compressive abilities in bone staples and its difficulty in manufacturability. Based on the description of the invention herein, those skilled in the art will realize the benefits of the current invention and will appreciate that the intent of this invention may be realized in other embodiments.

The present application and invention further includes the subject matter of the following numbered clauses:
1. An implant for osteosynthesis
   the implant having a first configuration when it is releasably attached to a delivery instrument and a second configuration when it is not attached to a delivery instrument, the implant or a portion thereof being comprised of a material that allows the implant to have multiple configurations,
   the implant having non-symmetrical means for fixation to bone segments, means to prevent or minimize rotation or other movement of the bone segments relative to one another and means to resist pullout of the implant from the bone segments.
2. The implant of clause 1 further comprising means for releasably engaging the delivery instrument.
3. The implant of clause 1 having two means for fixation to bone segments wherein the second configuration causes the means for fixation to compress, distract or control spacial orientation of the bone segments relative to one another.
4. The implant of clause 1 wherein the means for fixation are adapted to cause an interference fit with the bone segments.
5. The implant of clause 1 wherein the non-symmetrical means for fixation to bone segments are leg members.
6. The implant of clause 5 wherein the means to prevent or minimize rotation or other movement of the bone segments relative to one another comprise fin members or projections incorporated on the leg members.
7. The implant of clause 5 wherein the leg members comprise means for releasably engaging the delivery instrument.
8. The implant of clause 7 wherein the means for releasably engaging the delivery instrument comprise non-circular elongated openings extending through the legs.
9. The implant of clause 8 wherein the non-circular openings are in the shape of a tear drop, square, rectangle, oval, slot or keyhole.
10. The implant of clause 5 wherein the leg members comprise barbs configured to minimize or prevent pullout and/or rotation.
11. The implant of clause 5 wherein the leg members have tapered tips configured to facilitate insertion into bone.
12. The implant of clause 5 wherein the leg members are connected by a bridge member and the bridge member may or may not be of uniform thickness.
13. The implant of clause 8 wherein the leg members may or may not be of uniform thickness.
14. The implant of clause 8 wherein the non-circular elongated openings may or may not be of uniform cross section along the lengths thereof.
15. The implant of clause 5 wherein the leg members may or may not have a uniform cross section along the lengths thereof.
16. A kit comprising the implant of clause 1 and further comprising drills, drill guides, reamers, broaches and/or an inserter.
17. The kit of clause 16 comprising a sterile package.
18. The kit of clause 16 wherein the implant is releasably attached to the inserter.
19. The kit of clause 16 where in the implant is releasably attached to a holding apparatus for attaching to an inserter assembly.
20. A method of implanting the implant of clause 1 comprising the sequential steps of arranging the implant on a surgical site comprising bone segments, the implant being releasably engaged with a delivery instrument, affixing the implant in the bone segments and then releasing the implant from the delivery instrument.
21. The method of clause 19 wherein the implant comprises legs and holes are pre-drilled into the bone segments prior to arranging the implant on the surgical site wherein the holes are sized and/or shaped to receive the legs in an interference fit.
22. An assembly for delivering an implant to a surgical site for osteosynthesis comprising:
   an implant having a first configuration when it is releasably attached to a delivery instrument and a second configuration when it is not attached to the delivery instrument, the implant or a portion thereof being comprised of a material that allows the implant to have multiple configurations,
   the implant having non-symmetrical means for fixation to bone segments, means to prevent or minimize rotation or other movement of the bone segments relative to one another and means to resist pullout of the implant from the bone segments.
23. The assembly of clause 21 wherein the implant in the second configuration provides compression and/or distraction and/or control of spacial orientation when it is affixed to two or more than two bone members.
24. The assembly of clause 21 wherein the delivery instrument has a fixed engagement means for releasably engaging the implant.
25. The assembly of clause 23 wherein the fixed engagement means is comprised of parallel legs which force the implant into the first configuration.
26. The assembly of clause 21 wherein the delivery instrument has a means for ejecting the implant.
27. The assembly of clause 25 wherein the means for ejecting is an ejector pin.
28. The assembly of clause 21 wherein the delivery instrument has a
   connection means with the implant, the connection means being elongated to maintain the implant in the first configuration for insertion into bone or other tissue.

## Claims

1. An implant (300) for osteosynthesis,
the implant (300) having a first configuration when it is releasably attached to a delivery instrument (2000) and a second configuration when it is not attached to the delivery instrument (2000), the implant (300) or a portion thereof being comprised of a material that allows the implant (300) to have multiple configurations,
the implant (300) having first and second leg members (320) for engaging the bone, a bridge (310), a connection region between each leg member (320) and the bridge (310), and means to resist pullout of the implant (300) from the bone segments;
wherein the bridge (310) has a centre thickness (360), a first outer thickness (370) and a second outer thickness (380).;wherein the first outer thickness (370) is proximate the first leg member (320) and the second outer thickness (380) is proximate the second leg member (320).

2. The implant of claim 1, wherein the centre thickness (360) is greater than each of the first outer thickness (370) and the second outer thickness (380).

3. The implant of claims 1 or 2, wherein the centre thickness (360), the first outer thickness (370) and the second outer thickness (380) are each greater than the thickness of the connection regions connecting the bridge (310) and leg members (320).

4. The implant of any of claims 1 to 3, wherein the implant (300) further comprises means for releasably engaging the delivery instrument (2000).

5. The implant of any of claims 1 to 4, wherein the second configuration causes the leg members (320) to compress, distract or control spatial orientation of the bone segments relative to one another.

6. The implant of any preceding claim, wherein the leg members (320) are adapted to cause an interference fit with the bone segments; and/or
wherein a projection extends from an external surface of one of the leg members (320).

7. The implant of any preceding claim, wherein the leg members (320) comprise the means for releasably engaging the delivery instrument (2000).

8. The implant of claim 7, wherein the means for releasably engaging the delivery instrument (2000) comprise non-circular elongated openings extending through the leg members (320);
optionally wherein the non-circular elongated openings have at least one of: a teardrop shape, a square shape, a rectangle shape, an oval shape, a slot shape, and a keyhole shape;
further optionally wherein the non-circular elongated openings are of uniform cross-section.

9. The implant of any preceding claim, wherein the leg members (320) comprise barbs (330) configured to minimise or prevent rotation of the leg members (320) relative to the bone segments and resist pullout of the implant (300) from the bone segments.

10. The implant of any preceding claim, wherein the leg members (320) have tips that are tapered to facilitate insertion into bone.

11. The implant of any preceding claim, wherein the connection region has a geometry (350), optionally wherein the geometry is arched or gusseted.

12. A kit (1000) comprising an implant of any preceding claim and further comprising drills (1200), drill guides (1300), reamers, broaches and/or a delivery instrument (1500).

13. The kit of claim 12 comprising a sterile package; and/or
wherein the implant (300) is releasably attached to the delivery instrument (1500); and/or wherein the implant (300) is preassembled to the delivery instrument (1500).

14. An assembly for delivering an implant (300) to a surgical site for osteosynthesis comprising:
a delivery instrument (2000); and
an implant (300) of any of claims 1-11.

15. The assembly of claim 14 wherein the delivery instrument (2000) has a fixed engagement means for releasably engaging the implant (300);
optionally wherein the fixed engagement means is comprised of parallel legs which force the implant (300) into the first configuration; and/or wherein the delivery instrument (2000) has a means for ejecting the implant (300); optionally wherein the means for ejecting is an ejector pin (2020).
